# EUROPÄISCHE PATENTSCHRIFT

(11) **EP 1 663 382 B1**
(45) Veröffentlichungstag und Bekanntmachung des Hinweises auf die Patenterteilung: **29.04.2009**
(21) Anmeldenummer: 04766592.2
(22) Anmeldetag: 25.08.2004
(51) Int. Cl.: A61M 39/10, A61M 5/34, A61M 16/04

(54) **STECKBARE RÖHRENVERBINDUNG**
INSERTABLE PIPE CONNECTION
RACCORD DE TUYAUX ENFICHABLE

(30) Priorität: 04.09.2003 DE 10340708
(43) Veröffentlichungstag der Anmeldung: 07.06.2006
(73) Patentinhaber: TRACOE medical GmbH, 60528 Frankfurt am Main (DE)
(72) Erfinder: WALDECK, Franz, 55268 Nieder-Olm (DE)
(74) Vertreter: Weber, Roland
(86) Internationale Anmeldenummer: PCT/EP2004/051897
(87) Internationale Veröffentlichungsnummer: WO 2005/023360

(56) Entgegenhaltungen:
- US-A- 3 469 581
- US-A- 4 430 080
- US-A- 5 454 409
- US-A- 5 851 201

## Beschreibung

Die vorliegende Erfindung betrifft eine steckbare Röhrenverbindung mit einem ersten und einem zweiten röhrenförmigen Verbindungselement, wobei der Innendurchmesser des ersten Elements so groß ist, daß das zweite Elemente in das erste einschiebbar ist und wobei die innere Oberfläche des ersten und/oder die äußere Oberfläche des zweiten Verbindungselements im wesentlichen einen Konus mit einem hinreichend kleinen Konuswinkel bilden, so daß das erste mit dem zweiten Verbindungselement (im Bereich der Konusflächen) in kraftschlüssige, insbesondere reibschlüssige Verbindung bringbar ist.

Unter anderem im Bereich der Medizintechnik werden häufig Verbindungselemente benötigt, um Schlauchelemente oder Röhrenelemente, die zu medizinischen Versorgungseinrichtungen gehören, miteinander zu verbinden. Beispiele für derartige Verbindungen sind Kegelverbindungen mit einem 6% (Luer) Kegel für Spritzen, Kanülen und bestimmte andere medizinische Geräte wie der Anschluß eines Infusionsschlauchs an den Eingang eines Venenzugangs. Weitere Beispiele sind Anschlüsse an einen 15-mm Konnektor (15 mm Außenkegel) von Trachealtuben bzw. Tracheostomiekanülen für Atemschläuche von Anästhesiegeräten, entsprechende Schlauchadapter (T-Stücke, Y-Stücke), in Atemschläuche zwischengeschaltete Filterelemente oder sogenannte künstliche Nasen. Wichtige Anforderungen an solche Leitungsverbindungen stellen sich aus dem Klinikalltag heraus. So müssen die Verbindungen leicht herstellbar, dicht und einfach zu reinigen sein.

Die eingangs genannten und aus dem Stand der Technik bekannten steckbaren Röhrenverbindungen erfüllen diese Anforderungen weitgehend. Solche Röhrenverbindungen bestehen aus zwei zunächst getrennten Verbindungselementen, die an den Enden je eines Schlauches oder einer Röhre befestigt sind und selbst als im allgemeinen kurze Rohrstücke ausgestaltet sind, so daß Flüssigkeiten oder allgemein Fluide durch sie hindurchfließen können. Dabei sind die Durchmesser der Verbindungselemente so aufeinander abgestimmt, daß das zweite Element in enger Passung in das erste einschiebbar ist. Eine dichte und dauerhafte, wenn auch wieder lösbare Verbindung, wird dadurch erreicht, daß die innere Oberfläche des ersten und/oder die äußere Oberfläche des zweiten Verbindungselements im wesentlichen einen Konus mit einem hinreichend kleinen Konuswinkel bilden, so daß die beiden Verbindungselemente in kraft- bzw. reibschlüssige Verbindung bringbar sind. Dabei betragen die Konuswinkel der Verbindungselemente nur wenige Grad. In einer bevorzugten Ausführungsform sind die Konuswinkel der beiden Verbindungselemente im wesentlichen gleich. Durch die konusartige Ausgestaltung können Toleranzabweichungen in den Maßen der Verbindungselemente einfach aufgefangen werden in dem das zweite Element mehr oder weniger tief in das erste Element eingeschoben wird, bis sich eine kraftschlüssige und dichte Verbindung gebildet hat, bei der zumindest Teile der Innen- bzw. Außenflächen der Verbindungselemente direkt aufeinander liegen, so daß eine radiale Dichtfläche gebildet wird.

Die Europäische Norm EN 1281-1 : 1997 beschreibt entsprechende Verbindungselemente bzw. Konnektoren mit 8,5 mm, 15 mm, 22 mm, 23 mm und 30 mm Außenkegel.

Im folgenden wird im wesentlichen der medizinische Anwendungsbereich betrachtet, wobei es sich versteht, daß die Erfindung auch auf anderen Gebieten der Technik anwendbar ist, wo prinzipiell analoge Probleme auftreten können.

Bei den meisten der beschriebenen Verbindungselementen handelt es sich wie bei den Schläuchen und Röhren auch, um Einwegprodukte, die nach der zumeist einmaligen Verwendung entsorgt werden. Daher müssen die Verbindungselemente in großer Zahl zu niedrigen Kosten produziert werden. Sie werden zumeist aus preisgünstigem Kunststoff hergestellt. Dabei sind die Fertigungstoleranzen trotz moderner Herstellungsmethoden, zum Beispiel dem Spritzguß, verglichen mit Glas oder Metall groß. Aus diesem Grund können die Verbindungselemente nicht wie Glasstopfen unter vertretbarem Aufwand mit einem Einschliff versehen werden. Das sich daraus ergebende Problem ist, daß sich diese Verbindungselemente zwar unter Aufwendung moderater Kräfte gut zusammenstecken lassen und sich aufgrund der konischen Form auch eine dichte und dauerhafte Verbindung ergibt, jedoch sind sie häufig nur sehr schwer wieder lösbar. Dies ist auch eine Folge davon, daß der Gleitreibungskoeffizient zwischen den beiden Verbindungselementen aus Kunststoff beim Hereinschieben kleiner ist als der Haftreibungskoeffizient, der vorliegt, wenn die beiden Verbindungselemente statisch miteinander verbunden sind.

Dies ist zumeist solange kein Problem, wie die Röhrenverbindungen gut zugänglich sind und/oder vom medizinischen Personal getrennt werden. Notfalls können dann auch Hilfswerkzeuge verwendet werden. Soll jedoch der Patient selbst die Verbindung trennen, so können daraus Probleme erwachsen. Dies gilt insbesondere bei Verbindungselementen, die direkt am Körper an schlecht zugänglichen Stellen oder an Stellen, die ergonomisch nur schlecht handhabbar sind, liegen.

Ein Beispiel für eine solche Verbindung, bei der eine feste Verbindung der beiden Verbindungselemente, die vom Patienten selbst gelöst werden soll, zu Problemen, oft sogar zu Schmerzen führt, ist der Anschluß einer künstlichen Nase an den 15 mm Konnektor am Ende einer Tracheostomie-Kanüle. Das Ende der Tracheostomie-Kanüle mit dem als Konnektor bezeichneten zweiten Verbindungselement sitzt im Bereich des Kehlkopfes und ist für den Patienten nur mit stark angewinkelten Armen zugänglich. Beim Entfernen einer aufgesetzten Nase wird daher häufig, wenn die Verbindung zwischen Nase und Konnektor fest ist, ein Zug direkt auf die Kanüle ausgeübt, was für den Patienten unangenehm, schmerzhaft und gegebenenfalls schädlich sein kann.

Aus dem US-Patent 5,851,201 ist ein Luer-Verbinder bekannt, der verglichen mit einem herkömmlichen Luer-Verbinder eine erhöhte Eingriffskraft bereitstellt, sogar wenn ein kontaminierendes Fluid zwischen den Kontaktflächen der Verbindungselemente vorhanden ist. Der in der US 5,851,201 offenbarte Luer-Verbinder weist eine texturierte Kontaktfläche auf, die so angepasst ist, dass sie, kontaminierendes Fluid aufnimmt oder weg von den Kontaktflächen der passenden Luer-Anschlüsse führt.

Es ist daher Aufgabe der vorliegenden Erfindung, die aus dem Stand der Technik bekannte steckbare Röhrenverbindung so weiter zu entwickeln, daß sie leichter wieder lösbar und dennoch preiswert und dicht ist.

Diese Aufgabe wird, wie in Anspruch 1 dargelegt, dadurch gelöst, daß eine steckbare Röhrenverbindung mit einem ersten und einem zweiten röhrenförmigen Verbindungselement bereitgestellt wird, wobei der Innendurchmesser des ersten Elements so groß ist, daß das zweite Element in das erste einschiebbar ist und wobei die innere Oberfläche des ersten und/oder die äußere Oberfläche des zweiten Verbindungselements im wesentlichen einen Konus mit einem kleinen Konuswinkel bilden, so daß das erste mit dem zweiten Verbindungselement in kraftschlüssige, insbesondere reibschlüssige Verbindung bringbar ist, wobei mindestens eine der miteinander in Kontakt bringbaren Oberflächen der beiden Verbindungselemente oder Teile davon strukturiert sind, wobei die Oberflächenstruktur in Form von regel- oder unregelmäßig angeordneten Vertiefungen in einer ansonsten glatten konischen Oberfläche vorliegt und wobei die Grundflächen der Eindrückungen trapezförmig sind.

Die Strukturierung der miteinander in Eingriff tretenden Oberflächen des ersten und zweiten Verbindungselements bedeutet, daß die Oberfläche Vertiefungen und/oder Erhebungen auf der ansonsten glatten Konusfläche aufweist, und bewirkt, daß die Größe der effektiv unter einem größeren Kontaktdruck miteinander in Berührung kommenden Flächen verringert wird. Dadurch werden u. a. die Haftreibungskräfte reduziert und die Verbindung läßt sich leichter wieder lösen. Bei der Strukturierung ist jedoch darauf zu achten, daß die Strukturen dennoch keinen Leckdurchgang bilden, wobei sich vorzugsweise in Umfangsrichtung zusammenhängende Oberflächenbereiche ergeben, die einen dichten Abschluß bilden, so daß in axialer Richtung keine zusammenhängenden Kanäle entstehen, die Undichtigkeiten der steckbaren Röhrenverbindung nach sich ziehen. Durch das Ausbilden sehr flacher Strukturen, d. h. Vertiefungen geringer Tiefe oder Erhöhungen geringer Höhe kann man jedoch gegebenenfalls auch erreichen, daß zwar eine Berührung oder zumindest eine extrem schmaler Spalt auch im Bereich der Vertiefungen bzw. neben den Erhebungen auftritt, der eine dichte Verbindung gewährleistet, wobei jedoch der Kontaktdruck in diesen Bereichen gegenüber den verbleibenden konischen Kontaktflächen erheblich reduziert ist.

Bevorzugt wird eine Ausführungsform der Röhrenverbindung, bei der der Konuswinkel weniger als 20°, vorzugsweise weniger als 10° und besonders bevorzugt weniger als 5° beträgt. Dies ist zweck- mäßig, da bei Konuswinkeln von über 20° die Kräfte der reibschlüssigen Verbindung zwischen den beiden Verbindungselementen so weit reduziert sind, daß mit den gängigen Materialien keine dauerhaft feste und dichte Verbindung mehr hergestellt werden kann.

Zweckmäßig ist es, wenn die Oberflächenstruktur in Form von linienförmigen Eindrückungen und/oder Erhebungen vorliegt. Solche Eindrückungen oder Erhebungen, die regel- oder unregelmäßig auf der konusförmigen Oberfläche des ersten oder zweiten Verbindungselements angeordnet sind, reduzieren die Größe der sich berührenden Flächen erheblich. Wichtig für die Dichtigkeit der Verbindung ist, daß zumindest ein geschlossener Dichtungspfad in Umfangs-Richtung um das Verbindungselement herum besteht, so daß keine axialen Kanäle in der Röhrenverbindung gebildet werden, durch die das Fluid austreten kann, wobei im Falle viskoser Flüssigkeiten, wie sie beispielsweise in Form von Körpersekreten insbesondere in medizinischen Anwendungen häufig auftreten, enge bzw. schmale axiale Kanäle durchaus toleriert werden können, da entsprechend viskose Flüssigkeit nicht in nennenswertem Umfang durch genügend enge oder schmale Kanäle hindurchtreten. Dieser Effekt wird insbesondere in einer bevorzugten Ausführungsform der Erfindung genutzt, die axial verlaufende Vertiefungen in einer der konischen Flächen aufweist und dadurch besonders leicht zu lösen ist, ohne daß der Zusammenhalt der Verbindung im normalen Gebrauch beeinträchtigt ist.

Alternativ dazu kann die Oberflächenstruktur aus einer ringförmigen Auskragung aus der Oberfläche bestehen. Eine solche Ausgestaltung ist vorteilhaft, da die ringförmige Auskragung aus der Oberfläche eine Dichtlippe zwischen den beiden in Eingriff tretenden Verbindungselementen bildet. Wird die Auskragung dünn und flexibel ausgeführt, so läßt sich die steckbare Röhrenverbindung sehr leicht lösen, weist jedoch trotzdem die erforderliche Dichtigkeit auf.

Zweckmäßig ist es, wenn der Außendurchmesser des zweiten Verbindungselements an seinem vorderen Ende 15 mm beträgt. Eine solche Ausgestaltung des zweiten Verbindungselements ist kompatibel zu den 15-mm Normkonnektoren nach der Europäischen Norm EN 1281-1 : 1997.

Besonders bevorzugt ist eine Ausführungsform der Erfindung, bei der sich die Vertiefungen im wesentlichen in axialer Richtung, d.h. parallel zur Symmetrieachse der Röhre erstrecken. Nach den obigen Erläuterungen ermöglichen auch solche Vertiefungen unter der Voraussetzung, daß die Tiefe der Eindrückungen gering ist, eine dichte Verbindung zwischen den beiden Verbindungselementen der Röhrenverbindung, wobei die Verbindung aufgrund der verringerten Kontaktflächen, die einen höheren Kontaktdruck aufweisen, leicht lösbar ist.

Dabei ist es zweckmäßig, wenn die innere Oberfläche des ersten Verbindungselements mit Eindrükkungen bzw. Vertiefungen versehen ist und der äußere Durchmesser des zweiten Verbindungselement so bemessen ist, daß das erste Verbindungselement beim Einschieben des zweiten Verbindungselements aufgeweitet bzw, elastisch verformt wird, so daß eine reibschlüssige und dichte Verbindung zwischen den beiden Verbindungselementen entsteht.

Dabei ist es zweckmäßig, wenn die Grundflächen der Eindrückungen trapezförmig sind, d.h. vorzugsweise ein gleichschenkliges Trapez, ein Parallelogramm oder ein Rechteck bilden. Dabei ist es vorteilhaft, wenn zwei der Innenwinkel der Grundfläche der Eindrückungen dem Konuswinkel des Verbindungselements entsprechen. Auf diese Weise ergibt sich eine für eine feste und dichte Verbindung zwischen den beiden Verbindungselementen optimierte Geometrie.

Bevorzugt ist dabei eine Ausführungsform der Erfindung, bei der die Eindrückungen eine Länge von weniger als 15 mm, vorzugsweise weniger als 12 mm und insbesondere von etwa 9 mm aufweisen.

Dabei ist es zweckmäßig, wenn die Breite der Eindrückungen weniger als 5 mm und vorzugsweise weniger als 3,5 mm beträgt.

Die mögliche und bevorzugte Tiefe der Eindrückungen bzw. Vertiefungen hängt teilweise von ihrer Form und Orientierung ab. In manchen Ausführungsformen könnte die Tiefe bzw. Höhe der Strukturen ohne weiteres 1mm oder mehr betragen, in anderen Ausführungsformen, die z. B. sich axial erstreckende Strukturen aufweisen, muß die Tiefe womöglich geringer sein als 0,5 mm und kann insbesondere zwischen 0,05 und 3mm, z. B. 2 mm betragen.

Besonders vorteilhaft ist eine Ausführungsform der vorliegenden Erfindung, bei der nur die innere Oberfläche des ersten Verbindungselements oder nur die äußere Oberfläche des zweiten Verbindungselements strukturiert ist. Eine solche einseitige Strukturierung hat den Vorteil, daß nur eines der Werkzeuge zur Herstellung der Verbindungselements modifiziert werden muß und daß keine axialen Verbindungskanäle durch die Röhrenverbindung aus der Kombination der Oberflächenstrukturen der beiden Verbindungselemente entstehen können.

Bevorzugt wird eine Ausführungsform der Erfindung, bei der das zweite Verbindungselement den 15 mm-Konnektor einer Tracheostomie-Kanüle bildet. Gerade für solche Kanülen ist die erfindungsgemäße steckbare Röhrenverbindung vorteilhaft.

Auch ist es vorteilhaft, wenn das erste Verbindungselement als Anschluß an einer künstlichen Nase befestigt ist, da gerade bei einer künstlichen Nase die erfindungsgemäße Ausgestaltung einer steckbaren Röhrenverbindung die oben genannten Probleme löst.

Weitere Merkmale, Vorzüge und Anwendungsmöglichkeiten der vorliegenden Erfindung ergeben sich aus der nachfolgenden Beschreibung einer bevorzugten Ausführungsform zusammen mit den beiliegenden Figuren:
- Figur 1: zeigt eine seitliche Schnittansicht einer Röhrenverbindung,
- Figur 2: zeigt drei Ausführungsformen der inneren Oberflächen eines Verbindungselements, und
- Figur 3: zeigt eine Ausführungsform der erfindungsgemäßen Röhrenverbindung.

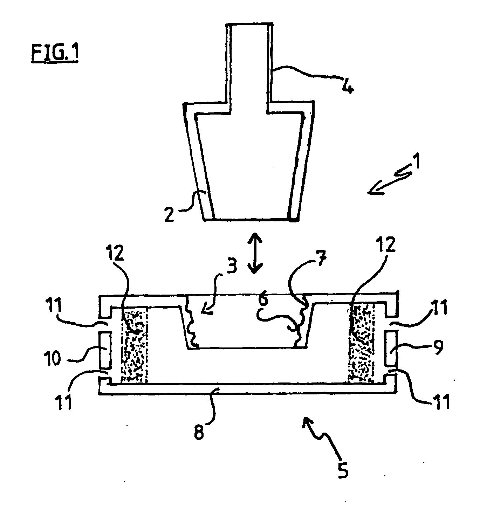

Figur 1 zeigt eine seitliche Schnittansicht einer Ausführungsform einer steckbaren Röhrenverbindung 1, bestehend aus einem ersten röhrenförmigen Verbindungselement 3 und einem zweiten röhrenförmigen Verbindungselement 2. Das zweite Verbindungselement 2, das hier in einer vergrößerten Ansicht dargestellt ist, entspricht einem aus der Medizintechnik bekannten Standardkonnektor mit 15 mm Durchmesser am vorderen Ende des Verbindungselements. Die äußere Oberfläche des Verbindungselements bildet einen Konus mit einem Konuswinkel < 10°. Am hinteren Ende des zweiten Verbindungselements 2 ist eine Reduzierung des Durchmessers des röhrenförmigen Verbindungselement 2 vorgesehen. Es schließt sich ein gerader Rohrabschnitt 4 mit verringertem Durchmesser an, auf den ein Schlauchelement aufgeschoben werden kann.

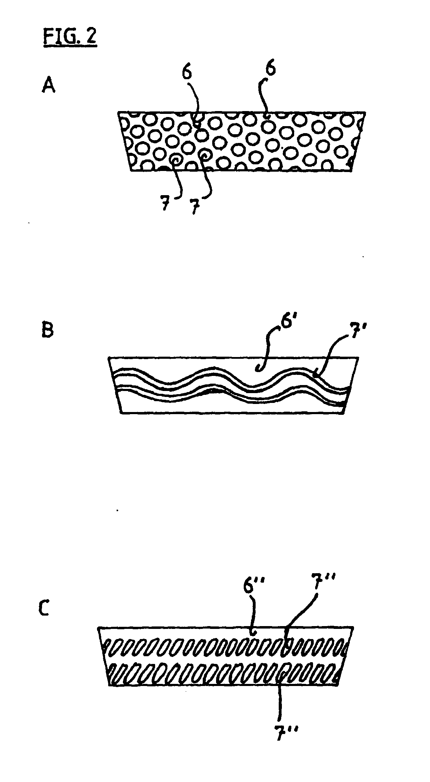

Der Außendurchmesser des zweiten Verbindungselements 2 ist im wesentlichen kleiner als der Innendurchmesser des ersten Verbindungselements 3, so daß das zweiten Verbindungselement 2 in das erste Verbindungselement 3 eingeschoben werden kann. Das erste Verbindungselement 3 ist in der dargestellten Ausführungsform mit einer künstlichen Nase 5 verbunden. Die innere Oberfläche 6 des ersten Verbindungselements 3 ist mit regelmäßig angeordneten Eindrückungen 7 versehen, die den Eindrückungen auf einem Golfball ähneln. Die Anordnung der Eindrückungen 7 auf der inneren Oberfläche 6 des ersten Verbindungselements 3 sind in ihrer regelmäßigen Anordnung besonders gut in Figur 2A zu erkennen.

Die künstliche Nase 5, mit der das erste Verbindungselement 3 fest verbunden ist, weist einen zylinderförmigen Hohlkörper 8 auf, der in seinen Deckelflächen 9, 10 Durchbrüche 11 zeigt, die den Innenraum des Hohlkörpers 8 mit der Umgebung verbinden. Das als künstliche Nase oder Wärme-und Feuchteaustauscher (HME) bezeichnet wird, arbeitet wie folgt. Die warme und feuchte Atemluft, die vom Patienten über eine Tracheostomie-Kanüle abgegeben wird, durchströmt von der Lunge her kommend zunächst das zweite Verbindungselement 2, das im Betriebszustand in die künstliche Nase 5 und das mit ihr verbundene erste Verbindungselement 3 eingeschoben ist. Die Atemluft strömt dann in den zylinderförmigen Hohlkörper 8 der künstlichen Nase 5 und gelangt durch die Filterelemente 12 und die Durchbrüche 11 in den Deckelflächen 9, 10 des zylinderförmigen Hohlkörpers 8 in die Umgebung. Die Filter 12 bestehen aus Filterpapier oder Schaumstoff, so daß sie einen Teil der Feuchtigkeit der Atemluft beim Ausatmen aufnehmen können, die sei beim Einatmen wieder an die einströmende Luft abgeben. Dadurch wird die einströmende Luft angefeuchtet und in gewissem Maß erwärmt und gefiltert. Somit übernimmt die künstliche Nase 5 Teile der Funktionen der menschlichen Nase beim Atmen.

Die Figuren 2A-C zeigen die abgerollte Mantelfläche der inneren Oberfläche 6, 6', 6" des ersten Verbindungselements 3 in drei verschiedenen Ausführungsformen. In Figur 2A ist die abgewickelte innere Oberfläche 6 gemäß der in der Figur 1 abgebildeten Ausführungsform des ersten Verbindungselements 3 gezeigt. Die Oberfläche 6 weist eine regelmäßige Anordnung von runden Eindrükkungen auf, wie sie von den Oberflächen von Golfbällen bekannt sind. In der gezeigten Ausführungsform beträgt der Durchmesser der Eindrückungen 7 in etwa 2 mm und ihre Tiefe in etwa 1 mm.

Die in Figur 2B gezeigte Ausführungsform der inneren Oberfläche 6' des ersten Verbindungselements 3' weist gewellte Eindrückungen 7' auf, die sich bandartig in Umfangsrichtung der Fläche 6' erstrecken. Die Breite der Eindrückungen 7' beträgt in der dargestellten Ausführungsform 1 mm.

Figur 2C zeigt eine dritte Ausführungsform der inneren Oberfläche 6" des ersten Verbindungselements 3". Die Eindrückungen 7" sind länglich fadenartig ausgebildet, so daß die Oberfläche 6" in Umfangsrichtung lamellenartig erscheint. Hervorzuheben ist, daß die Oberfläche 6" auch in dieser Ausführungsform auf der gesamten Länge des Umfangs zusammenhängende Abschnitte aufweist.

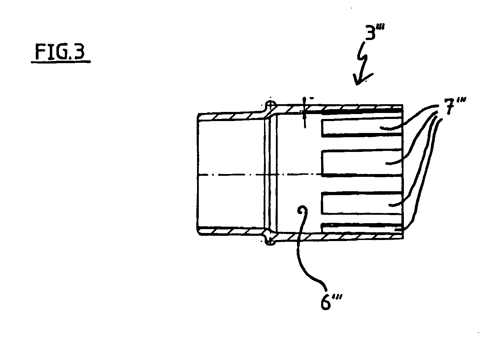

In Figur 3 ist eine erfindungsgemäße Ausführungsform des ersten Verbindungselements 3''' dargestellt. Die innere Oberfläche 6''' des ersten Verbindungselements 3''' weist in dieser Ausführungsform Eindrükkungen 7''' auf, welche sich in der ansonsten glatten und konisch zulaufenden inneren Oberfläche 6''' erstrecken. Die Eindrückungen 7''' erstrecken sich im wesentlichen parallel zur Symmetrieachse des Verbindungselements 3'''. Das heißt, sie haben eine größere Ausdehnung in Richtung der Durchflußrichtung des Verbindungselements 3''' als in der Richtung senkrecht dazu. Die dargestellten Eindrückungen 7''' bilden genau genommen gleichschenklige Trapeze, die aber nahezu Rechtecke sind, wobei der Winkel zwischen zwei Seiten eines solchen Trapezes einem Bruchteil d/π des Konuswinkels des Verbindungselements 3''' entspricht. Die Tiefe der Eindrückungen 7''' ist mit 0,2 mm so gewählt, daß sich eine Verringerung der unter einem höheren Kontaktdruck aufeinanderliegenden Oberflächen zwischen dem ersten und dem zweiten Verbindungselement ergibt und sich trotzdem auch im Bereich der Vertiefungen ein leichter Oberflächenkontakt oder zumindest eine ausreichende Reduzierung des Spaltes zwischen den einander gegenüber liegenden Flächen ergibt, so daß eine ausreichende Dichtigkeit erzielt wird. Beim Einschieben des zweiten, vorzugsweise konischen Verbindungselements in das erste Verbindungselement 3''' wird dieses ein wenig gedehnt und elastisch verformt, so daß auch die Flächen der Vertiefungen 7''' zumindest teilweise in Kontakt mit dem zweiten Verbindungselement treten und eine dichte Verbindung zwischen den beiden Verbindungselementen hergestellt wird. Idealerweise reicht die vordere Kante des vollständig eingeschobenen inneren Verbindungsteiles gerade bis an das axiale Ende der Vertiefungen des äußeren Verbindungsteiles heran oder geringfügig darüber hinaus. Man erreicht dies durch passende Abstimmung der Innen- und Außendurchmesser und des Konuswinkels der Verbindungselemente.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

## Patentansprüche

1. Steckbare Röhrenverbindung (1) mit einem ersten und einem zweiten röhrenförmigen Verbindungselement (3, 2), wobei der Innendurchmesser des ersten Elements (3) und der Außendurchmesser des zweiten Elements (2) so bemessen sind, daß das zweite Element (2) passend in das erste einschiebbar ist und wobei die innere Oberfläche (6, 6', 6") des ersten und/oder die äußere Oberfläche (6, 6', 6") des zweiten Verbindungselementes (2) im wesentlichen einen Konus mit einem kleinen Konuswinkel bilden, so daß das erste mit dem zweiten Verbindungselement (3, 2) in kraftschlüssige Verbindung bringbar ist, wobei mindestens eine der miteinander in Kontakt bringbaren Oberflächen (6, 6', 6") der beiden Verbindungselemente (2,3) oder Teile davon strukturiert sind, wobei die Oberflächenstruktur in Form von regel- oder unregelmäßig angeordneten Eindrückungen (7, 7', 7") vorliegt, **dadurch gekennzeichnet, daß** die Grundflächen der Eindrückungen trapezförmig sind.

2. Steckbare Röhrenverbindung (1) nach Anspruch 1, **dadurch gekennzeichnet, daß** der Konuswinkel weniger als 20° beträgt.

3. Steckbare Röhrenverbindung (1) nach Anspruch 1 oder 2, **dadurch gekennzeichnet, daß** die Eindrückungen (7, 7', 7") weniger als 2 mm bevorzugt tief sind.

4. Steckbare Röhrenverbindung (1) nach einem der Ansprüche 1 bis 3, **dadurch gekennzeichnet, daß** die Eindrückungen (7') nach Art einer Golfballoberfläche ausgeführt sind.

5. Steckbare Röhrenverbindung (1) nach einem der Ansprüche 1 bis 4, **dadurch gekennzeichnet, daß** der Außendurchmesser des zweiten Verbindungselements (2) an seinem vorderen Ende 15 mm beträgt.

6. Steckbare Röhrenverbindung (1) nach einem der Ansprüche 1 bis 5, **dadurch gekennzeichnet, daß** sich die Eindrückungen im wesentlichen in axialer Richtung erstrecken.

7. Steckbare Röhrenverbindung (1) nach einem der Ansprüche 1 bis 6, **dadurch gekennzeichnet, daß** die Grundflächen der Eindrückungen ein gleichschenkliges Trapez, ein Parallelogramm oder ein Rechteck bilden.

8. Steckbare Röhrenverbindung (1) nach einem der Ansprüche 1 bis 7, **dadurch gekennzeichnet, daß** der Winkel zwischen zwei Seiten der Grundfläche der Eindrückungen einem Bruchteil d/π des Konuswinkels entspricht, wobei d der Winkelumfang der Vertiefung in Radian ist.

9. Steckbare Röhrenverbindung (1) nach einem der Ansprüche 1 bis 8, **dadurch gekennzeichnet, daß** die Eindrückungen eine Länge von weniger als 10 mm aufweisen.

10. Steckbare Röhrenverbindung (1) nach Anspruch 9, **dadurch gekennzeichnet, daß** die Eindrückungen eine Länge von 9 mm aufweisen.

11. Steckbare Röhrenverbindung (1) nach einem der Ansprüche 1 bis 9, **dadurch gekennzeichnet, daß** die Breite der Eindrückungen weniger als 5 mm beträgt.

12. Steckbare Röhrenverbindung (1) nach Anspruch 11, **dadurch gekennzeichnet, daß** die Breite der Eindrückungen weniger als 3,5 mm beträgt.

13. Steckbare Röhrenverbindung (1) nach einem der Ansprüche 1 bis 12, **dadurch gekennzeichnet, daß** nur die innere Oberfläche (6, 6', 6") des ersten Verbindungselements (3) oder nur die äußere Oberfläche des zweiten Verbindungselements (2) strukturiert ist.

14. Steckbare Röhrenverbindung (1) nach einem der Ansprüche 1 bis 13, **dadurch gekennzeichnet, daß** das zweite Verbindungselement (2) als Konnektor an einer Tracheostomiekanüle oder einem Trachealtubus befestigt ist.

15. Steckbare Röhrenverbindung (1) nach einem der Ansprüche 1 bis 14, **dadurch gekennzeichnet, daß** das erste Verbindungselement (3) als Anschluß an einer künstlichen Nase (5) befestigt ist.

## Claims

1. An insertable pipe connection (1) comprising a first and second tubular connecting element (3, 2), the size of the inner diameter of the first element (3) and the outer diameter of the second element (2) being such that the second element (2) can be inserted into the first element, fitting therein, and the inner surface (6, 6', 6") of the first and/or outer surface (6, 6', 6") of the second connecting element (2) essentially form(s) a cone with a small conical angle such that the first connecting element can be connected to the second connecting element (3,2) in a non-positive, particularly frictionally engaged manner, at least one of the interconnectable surfaces (6, 6', 6") of the two connecting elements (2, 3) or parts thereof being structured, wherein the surface structure is provided in the form of regularly or irregularly arranged indentations (7, 7', 7"), **characterized in that** the bases of the indentations are trapezoidal in shape.

2. An insertable pipe connection (1) according to claim 1, **characterized in that** the conical angle is less than 20°.

3. An insertable pipe connection (1) according to claim 1 or 2, **characterized in that** the indentations (7, 7', 7") have a depth of preferably less than 2 mm.

4. An insertable pipe connection (1) according to one of claims 1 to 3, **characterized in that** the indentations (7') are designed in a golf ball surface-like manner.

5. An insertable pipe connection (1) according to one of claims 1 to 4, **characterized in that** the outer diameter of the second connecting element (2) is 15 mm at its front end.

6. An insertable pipe connection (1) according to one of claims 1 to 5, **characterized in that** the indentations substantially extend in an axial direction.

7. An insertable pipe connection (1) according to one of claims 1 to 6, **characterized in that** the bases of the indentations form an equal-sided trapezoid, a parallelogram or a rectangle.

8. An insertable pipe connection (1) according to one of claims 1 to 7, **characterized in that** the angle between two sides of the basis of the indentations corresponds to a fractional amount d/π of the conical angle, wherein d is the angular extension of the recess, measured in radian.

9. An insertable pipe connection (1) according to one of claims 1 to 8, **characterized in that** the indentations have a length of less than 10 mm.

10. An insertable pipe connection (1) according to claim 9, **characterized in that** the indentations have a length of 9 mm.

11. An insertable pipe connection (1) according to one of claims 1 to 9, **characterized in that** the width of the indentations is less than 5 mm.

12. An insertable pipe connection (1) according to claim 11, **characterized in that** the width of the indentations is less than 3.5 mm.

13. An insertable pipe connection (1) according to one of claims 1 to 12, **characterized in that** only the inner surface (6, 6', 6") of the first connecting element (3) or only the outer surface of the second connecting element (2) is structured.

14. An insertable pipe connection (1) according to one of claims 1 to 13, **characterized in that** the second connecting element (2) is fastened to a tracheotomy cannula or a tracheal tube as a connector.

15. An insertable pipe connection (1) according to one of claims 1 to 14, **characterized in that** the first connecting element (3) is fastened to an artificial nose (5) as a connector.

## Revendications

1. Raccord de tuyaux enfichable (1) comprenant un premier et un second éléments de raccord tubulaires (3, 2), le diamètre intérieur du premier élément (3) et le diamètre extérieur du second élément (2) étant dimensionnés de sorte que le second élément (2) peut être introduit de façon ajustée dans le premier et la surface intérieure (6, 6', 6") du premier élément de raccord (2) et/ou la surface extérieure (6, 6', 6") du second élément de raccord (2) formant sensiblement un cône doté d'un angle réduit, de sorte que le premier élément de raccord (3) peut être amené en raccordement par coopération de forces avec le second élément de raccord (2), au moins une des surfaces (6, 6', 6"), pouvant être amenées en contact les unes avec les autres, des deux éléments de raccordement (2, 3) ou ses parties étant structurées, la structure de la surface se présentant sous forme d'empreintes (7, 7', 7") disposées régulièrement ou irrégulièrement, **caractérisé en ce que** les surfaces de base des empreintes sont trapézoïdales.

2. Raccord de tuyaux enfichable (1) selon la revendication 1, **caractérisé en ce que** l'angle du cône mesure moins de 20°.

3. Raccord de tuyaux enfichable (1) selon la revendication 1 ou la revendication 2, **caractérisé en ce que** les empreintes (7, 7', 7") présentent une profondeur de préférence inférieure à 2 mm.

4. Raccord de tuyaux enfichable (1) selon l'une quelconque des revendications 1 à 3, **caractérisé en ce que** les empreintes (7') sont réalisées comme la surface d'une balle de golf.

5. Raccord de tuyaux enfichable (1) selon l'une quelconque des revendications 1 à 4, **caractérisé en ce que** le diamètre extérieur du second élément de raccord (2) à son extrémité avant mesure 15 mm.

6. Raccord de tuyaux enfichable (1) selon l'une quelconque des revendications 1 à 5, **caractérisé en ce que** les empreintes s'étendent sensiblement dans la direction axiale.

7. Raccord de tuyaux enfichables (1) selon l'une quelconque des revendications 1 à 6, **caractérisé en ce que** les surfaces de base des empreintes forment un trapèze isocèle, un parallélogramme ou un rectangle.

8. Raccord de tuyaux enfichable (1) selon l'une quelconque des revendications 1 à 7, **caractérisé en ce que** l'angle compris entre deux faces de la surface de base des empreintes correspond à une fraction d/π de l'angle du cône, d étant le périmètre angulaire du creux en radian.

9. Raccord de tuyaux enfichable (1) selon l'une quelconque des revendications 1 à 8, **caractérisé en ce que** les empreintes présentent une longueur inférieure à 10 mm.

10. Raccord de tuyaux enfichable (1) selon la revendication 9, **caractérisé en ce que** les empreintes présentent une longueur de 9 mm.

11. Raccord de tuyaux enfichable (1) selon l'une quelconque des revendications 1 à 9, **caractérisé en ce que** la largeur des empreintes mesure moins de 5 mm.

12. Raccord de tuyaux enfichable (1) selon la revendication 11, **caractérisé en ce que** la largeur des empreintes mesure moins de 3,5 mm.

13. Raccord de tuyaux enfichable (1) selon l'une quelconque des revendications 1 à 12, **caractérisé en ce que** seule la surface intérieure (6, 6', 6") du premier élément de raccord (3) ou seule la surface extérieure du second élément de raccord (2) est structurée.

14. Raccord de tuyaux enfichable (1) selon l'une quelconque des revendications 1 à 13, **caractérisé en ce que** le second élément de raccord (2) est fixé en tant que connecteur à une canule de trachéotomie ou à un tube trachéal.

15. Raccord de tuyaux enfichable (1) selon l'une quelconque des revendications 1 à 14, **caractérisé en ce que** le premier élément de raccord (3) est fixé en tant que raccordement à un nez artificiel (5).
